# EUROPEAN PATENT APPLICATION

(11) **EP 4 775 131 A1**
(43) Date of publication of application: **15.07.2026**
(21) Application number: 24863183.0
(22) Date of filing: 04.09.2024
(51) Int. Cl.: A61B 5/00, A61B 5/08, G16H 50/20, G16H 50/30

(54) **APPARATUS AND METHOD FOR DETERMINING STATE OF PULMONARY CONGESTION BY USING ARTIFICIAL INTELLIGENCE ANALYSIS**

(30) Priority: 08.09.2023 KR 20230119664; 07.02.2024 KR 20240019014
(71) Applicant: A.Cure, Seoul 08514 (KR)
(72) Inventor: KIM, Eung Ju, Seoul 08096 (KR); KO, Hanseok, Seoul 04426 (KR); LEE, Ji Eun, Seoul 03710 (KR); KIM, Gwan Tae, Seoul 01619 (KR); KO, Kyung-Deuk, Hanam-si Gyeonggi-do 12946 (KR); HAM, In Sung, Goyang-si Gyeonggi-do 10584 (KR)
(74) Representative: Caspary, Karsten
(86) International application number: PCT/KR2024/013317
(87) International publication number: WO 2025/053603

(57) **Abstract**

Disclosed are an apparatus and a method for determining the state of pulmonary congestion by using artificial intelligence analysis. The apparatus for determining the state of pulmonary congestion by using artificial intelligence analysis, according to an embodiment, may comprise: a data reception unit that receives speech data from a user; a data preprocessing unit that extracts, from the received speech data, partial speech data corresponding to a portion of the speech data by using windowing processing, and converts the extracted partial speech data into a spectrogram; and a pulmonary congestion state determination unit that outputs, by using the spectrogram and a neural network-based pulmonary congestion state determination model, determination data including pulmonary congestion state information about the user.

## Description

### [Technical Field]

The following disclosure relates to an apparatus and a method for determining a state of pulmonary congestion by using artificial intelligence analysis.

### [Background Art]

Technologies for differentiating pulmonary congestion due to heart failure include blood biomarkers (for example, B-type natriuretic peptide (BNP), N-terminal pro-BNT (NT-proBNP), growth stimulation expressed gene 2 (ST2)), lung imaging (for example, chest X-ray, chest Computed Tomography (CT)), and Cadio Micro-Electro-Mechanical Systems (MEMS), which monitors pulmonary artery pressure through a sensor inserted into the pulmonary artery. Fluid overload from acute heart failure may cause swelling in the lungs, larynx, and articulatory organs (for example, vocal cords, tongue, palate, and mouth muscles), which are the main organs that produce voice through the flow of air.

### [Technical Solution]

An apparatus for determining a state of pulmonary congestion by using artificial intelligence analysis according to an embodiment includes a data reception unit configured to receive speech data from a user, a data preprocessing unit configured to extract partial speech data corresponding to a portion of the received speech data by using windowing processing and to convert the extracted partial speech data into a spectrogram, and a pulmonary congestion state determination unit configured to output determination data including pulmonary congestion state information of the user by using a neural network-based pulmonary congestion state determination model and the spectrogram.

The pulmonary congestion state determination model may be a dense network including a convolution layer, a dense layer, a transition layer, and a classification layer.

The pulmonary congestion state determination model may be a dense network and may be configured to extract hidden features through skip-connections of the dense network and to determine the pulmonary congestion state information by using the extracted hidden features.

An output of the pulmonary congestion state determination model may include at least one of a probability value corresponding to a state in which pulmonary congestion of the user has improved and a probability value corresponding to a state in which pulmonary congestion of the user has not improved.

The pulmonary congestion state determination model may be a vision transformer, and the pulmonary congestion state determination model may include a projection unit configured to convert a dimension of a feature vector of the spectrogram, a transformer encoder, and a classification layer.

The data preprocessing unit may be configured to divide the spectrogram into a plurality of partial spectrograms and to input at least one of the divided partial spectrograms into the vision transformer.

The vision transformer may be configured to perform vector projection on the divided partial spectrogram, calculate attention of the vector-projected partial spectrogram, and determine pulmonary congestion state information based on the calculated attention.

The speech data from the user may be speech data from a patient suffering from pulmonary congestion due to acute heart failure.

The spectrogram may be a mel-spectrogram represented as a graph in which speech data of the user is transformed into frequency information corresponding to the speech data by a Fourier transform, and a scale of the frequency information may be converted into a mel-scale.

The spectrogram may be input to the pulmonary congestion state determination model, and at least one of user information of the user and blood test information of the user may be further input to the pulmonary congestion state determination model.

A method for determining a state of pulmonary congestion performed by an apparatus for determining a state of pulmonary congestion according to an embodiment includes receiving speech data from a user by using a data reception unit, extracting partial speech data corresponding to a portion of the received speech data by using windowing processing and converting the extracted partial speech data into a spectrogram, and obtaining determination data including pulmonary congestion state information of the user by using a neural network-based pulmonary congestion state determination model and the spectrogram.

In the method for determining a state of pulmonary congestion, the spectrogram may be divided into a plurality of partial spectrograms, and at least one of the divided partial spectrograms may be input to the vision transformer.

### [Description of Drawings]

FIG. 1 is a block diagram for describing the configuration of an apparatus for determining the state of pulmonary congestion according to an embodiment.
FIG. 2 is a view for describing preprocessing of speech data by using a data preprocessing unit according to the embodiment.
FIG. 3 is a view for describing a pulmonary congestion state determination model according to the embodiment.
FIG. 4 is a view for describing the pulmonary congestion state determination model according to the embodiment.
FIG. 5 is a flowchart for describing the operations of a method for determining a state of pulmonary congestion according to an embodiment.

### [Modes of the Invention]

Specific structural or functional descriptions of the embodiments are disclosed for illustrative purposes only and may be modified and implemented in various forms. Therefore, the actual implementation is not limited to the specific embodiments disclosed, and the scope of the present specification includes modifications, equivalents, or alternatives within the technical concepts described in the embodiments.

Although terms such as "first" or "second" may be used to describe various components, these terms should be interpreted solely to distinguish one component from another. For example, a first component may be referred to as a second component, and similarly, a second component may also be referred to as a first component.

When it is said that a component is "connected" to another component, it should be understood that it may be directly connected or connected to that other component, but there may also be other components in between.

Singular expressions include plural expressions unless the context clearly dictates otherwise. In the present document, phrases such as "A or B", "at least one of A and B", "at least one of A or B", "A, B, or C", "at least one of A, B, and C", and "at least one of A, B, or C" may each include any one of the items listed in that phrase, or any and all possible combinations thereof. In the present specification, it should be understood that the terms "comprise" or "include" are intended to specify the presence of a described feature, number, step, operation, component, portion, or combination thereof, but do not preclude the presence or addition of one or more other features, numbers, steps, operations, components, portions, or combinations thereof.

Unless otherwise defined, all terms used herein, including technical or scientific terms, have the same meaning as commonly understood by a person of ordinary skill in the art. Terms defined in commonly used dictionaries should be interpreted to have a meaning consistent with their meaning in the context of the relevant technology, and will not be interpreted in an idealized or overly formal sense unless explicitly defined in the present specification.

The term "unit" used in the present document refers to a software or hardware component such as an FPGA or ASIC, and the "unit" performs certain functions. However, the "unit" is not limited to software or hardware. The "unit" may be configured to reside on an addressable storage medium and may be configured to execute one or more processors. For example, the "unit" may include components such as software components, object-oriented software components, class components, and task components, as well as processes, functions, attributes, procedures, subroutines, segments of program code, drivers, firmware, microcode, circuitry, data, databases, data structures, tables, arrays, and variables. The functionality provided within the components and "units" may be combined into a smaller number of components and "units" or further separated into additional components and "portions." Furthermore, the components and "units" may be implemented to execute one or more central process units (CPUs) in a device or a secure multimedia card. Additionally, 'portion' may include one or more processors.

Hereinafter, embodiments will be described in detail with reference to the attached drawings. In the description with reference to the attached drawings, the same components are denoted by the same reference numerals regardless of the drawing numbers, and redundant descriptions thereof will be omitted.

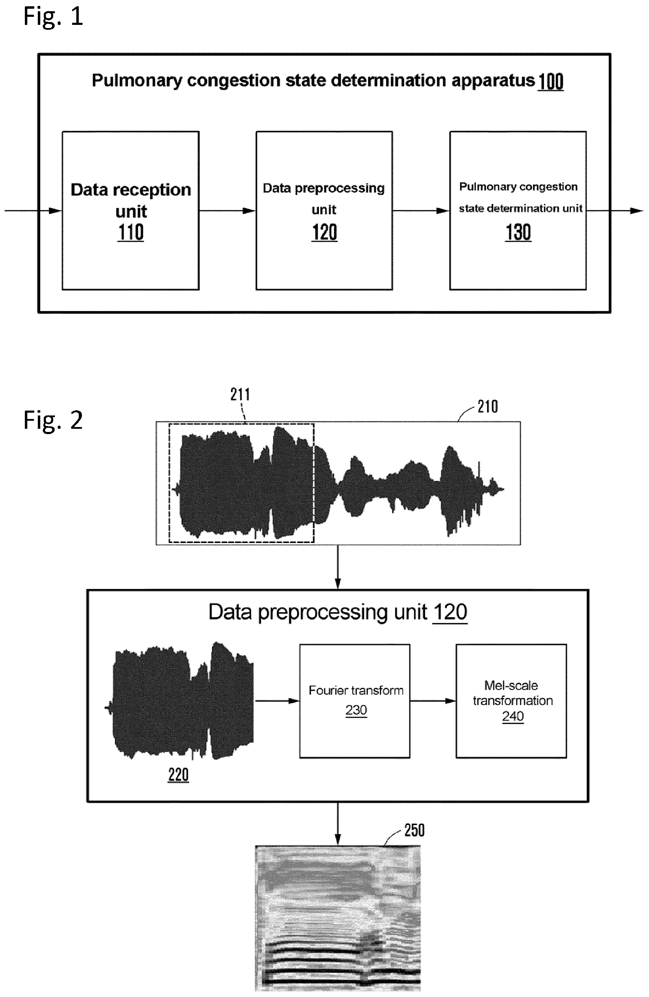

FIG. 1 is a block diagram for describing the configuration of an apparatus for determining the state of pulmonary congestion according to an embodiment.

Referring to FIG. 1, a pulmonary congestion state determination apparatus 100 is an apparatus that uses artificial intelligence analysis to determine a user's pulmonary congestion state. Pulmonary congestion indicates an increase in blood volume in the pulmonary blood vessels and congestion in the pulmonary circulation. According to an embodiment, the pulmonary congestion state determination apparatus 100 may include a data reception unit 110, a data preprocessing unit 120, and a pulmonary congestion state determination unit 130.

The data reception unit 110 may receive the user's speech data. The user speech data received by the data reception unit 110 may be speech data of a patient suffering from pulmonary congestion due to acute heart failure. For example, the speech data of the user may be data in which a patient suffering from pulmonary congestion due to acute heart failure pronounces "Daehanminguk Manse," "ah," "eh," "ee," "oh," and "oo." Pulmonary congestion due to acute heart failure indicates pulmonary congestion caused by structural or functional abnormalities of the heart.

The data preprocessing unit 120 may preprocess the user's speech data. In the embodiment, the data preprocessing unit 120 may extract partial speech data corresponding to a portion of the speech data from the received speech data by using windowing processing. The data preprocessing unit 120 may convert the extracted partial speech data into a spectrogram. The spectrogram is a graph that visualizes the sound spectrum. The preprocessing of the user's speech data by the data preprocessing unit 120 is described in detail with reference to FIG. 2.

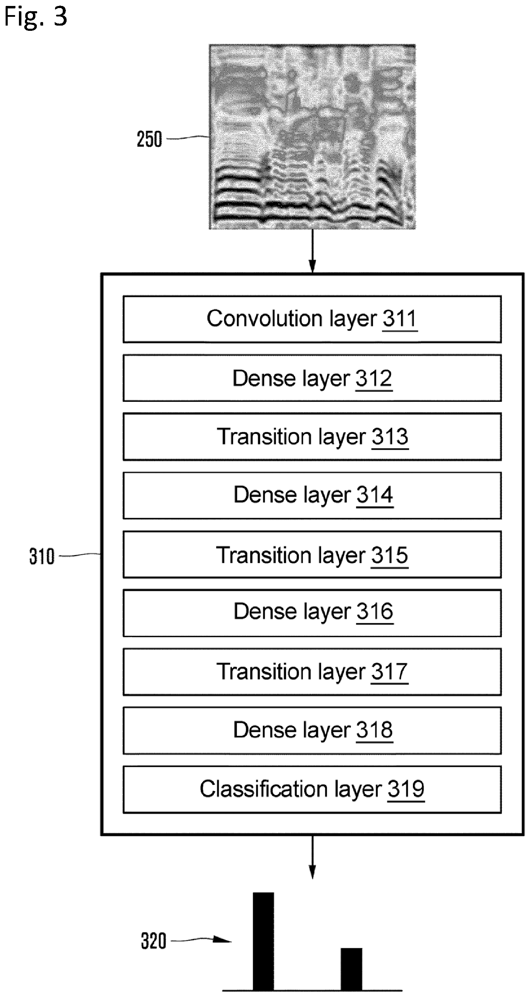

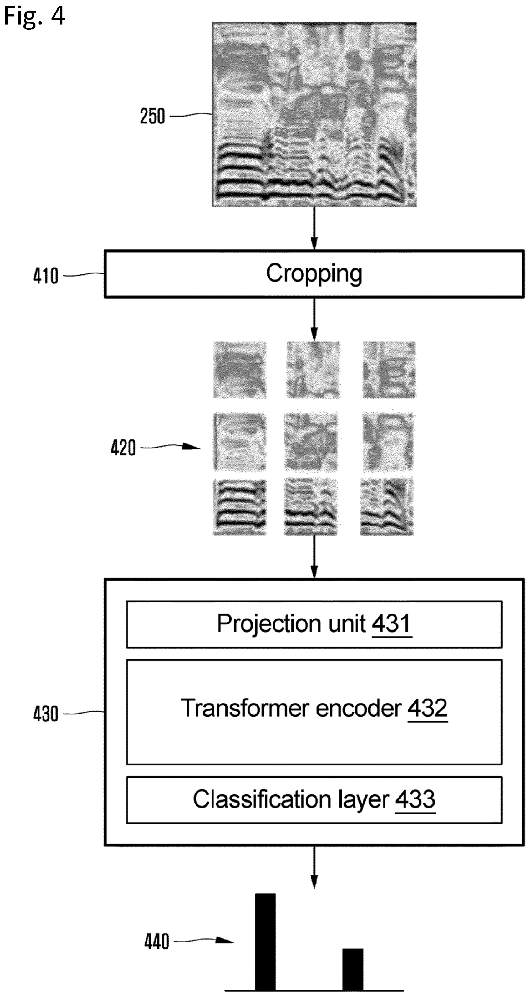

The pulmonary congestion state determination unit 130 may output determination data including pulmonary congestion state information. For example, the pulmonary congestion state determination unit 130 may output determination data including the user's pulmonary congestion state information using a neural network-based pulmonary congestion state determination model and the spectrogram. The pulmonary congestion state determination unit 130 that outputs determination data by using the neural network-based pulmonary congestion state determination model is described in detail with reference to FIGS. 3 and 4.

The pulmonary congestion state determination apparatus 100 according to the embodiment may distinguish the state of pulmonary congestion in patients with heart failure by using a pulmonary congestion determination model. The pulmonary congestion state determination apparatus 100 may determine and monitor the state of pulmonary congestion in patients with heart failure by using a negative biomarker for the state of pulmonary congestion. Unlike conventional pulmonary congestion diagnosis and monitoring technologies, the pulmonary congestion state determination apparatus 100 may not require blood analysis or X-ray photography. Unlike the CadioMEMS method in the related art, the pulmonary congestion state determination apparatus 100 may not require a visit to the hospital and may not require continuous monitoring and management by medical staff. Therefore, the pulmonary congestion state determination apparatus 100 has superior advantages over the CadioMEMS method in terms of cost, convenience, safety, and management.

Pulmonary congestion due to heart failure may cause respiratory distress and lead to hospitalization or death. Furthermore, pulmonary congestion may increase medical expenses and reduce the patient's quality of life. The pulmonary congestion state determination apparatus 100 according to the embodiment may improve the patient's quality of life by reducing mortality, readmission rates, and medical expenses due to heart failure. Furthermore, the pulmonary congestion state determination apparatus 100 is superior to existing techniques in the related art in that it can diagnose and monitor the state of pulmonary congestion due to heart failure at low cost by noninvasively recording his or her own voice anywhere.

FIG. 2 is a view for describing how a data preprocessing unit according to the embodiment preprocesses speech data.

Referring to FIG. 2, the data preprocessing unit 120 may preprocess speech data 210 to extract partial speech data 220 corresponding to a portion 211 of the speech data. The data preprocessing unit 120 according to the embodiment may output the partial speech data 220 by using windowing processing. For example, the data preprocessing unit 120 may apply a window having an interval of 1 second (or N (a natural number greater than or equal to 2) seconds) to the speech data 210 to extract partial speech data 220 corresponding to the area of the window.

According to the embodiment, the data preprocessing unit 120 may convert the partial speech data 220 into data in the frequency domain. For example, the data preprocessing unit 120 may convert the partial speech data 220 into frequency information by performing a Fourier transform 230 on the partial speech data 220.

The data preprocessing unit 120 may perform a mel-scale transformation 240 on the frequency information of partial speech data converted into frequency domain data to generate a mel-spectrogram 250. The mel-scale is a scale that reflects the standard for human perception of sound, and the mel-spectrogram 250 is a spectrogram that includes the frequency information of the mel-scale.

In the embodiment, the mel-spectrogram 250 preprocessed by the data preprocessing unit 120 may be input to a pulmonary congestion state determination model. According to the embodiment, the pulmonary congestion state determination model may include a resnet (for example, resnet-50, resneXt), a densenet 310 (for example, densenet), a convnet (for example, ConvnetXt), an efficientnet (for examle, efficientnet-b7), and/or a vision transformer (ViT).

The pulmonary congestion state determination model according to the embodiment may output a binary classification value (for example, binary classification value 320 of FIG. 3, binary classification value 440 of FIG. 4) based on the input mel-spectrogram 250. For example, the pulmonary congestion state determination model may output a binary classification value including at least one of a probability value corresponding to an improved state of the user's pulmonary congestion and a probability value corresponding to an unimproved state of the user's pulmonary congestion by using the preprocessed mel-spectrogram 250.

According to the embodiment, the pulmonary congestion state determination model may further input at least one of user information and blood test information of a user in addition to a spectrogram (for example, Mel-spectrogram 250). The user information is personal information about a user who uses the apparatus for determining a state of pulmonary congestion and may include, for example, age, gender, medical history, and/or lifestyle information. The blood test information is medical information about the user obtained by testing the user's blood and may include, for example, information about B-type natriuretic peptide (BNP), N-terminal pro-BNT (NT-proBNP), and/or growth stimulation expressed gene 2 (ST2).

FIG. 3 is a view for describing how DensNet outputs binary classification values according to the embodiment.

Referring to FIG. 3, the apparatus for determining a state of pulmonary congestion (for example, pulmonary congestion state determination apparatus 100 of FIG. 1) may determine a pulmonary congestion state using a dense network 310 as a pulmonary congestion state determination model. The dense network 310 may include a plurality of layers. The dense network 310 may include, for example, a convolution layer 311, dense layers 312, 314, 316, and 318, transition layers 313, 315, and 317, and a classification layer 319. Each of the convolution layer, the dense layers, the transition layers, and the classification layer included in the dense network 310 may be one or more.

The convolution layer 311 according to the embodiment may extract a feature vector of input data. For example, the convolution layer 311 may extract a feature vector from an input mel-spectrogram (for examle, mel-spectrogram 250 of FIG. 2).

According to the embodiment, the dense layers 312, 314, 316, and 318 represent densely connected layers. For example, the features extracted from the convolution layer 311 may be input to the transition layers 313, 315, and 317 through dense layers 312, 314, 316, and 318. According to the embodiment, the dense network 310 may extract the features related to pulmonary congestion state information through skip-connections. For example, the dense network 310 may extract hidden features through skip-connections between the layers. Skip-connections represent a method in which an input layer skips at least one of the intermediate layers and is directly connected to the output layer.

The transition layers 313, 315, and 317 according to the embodiment may reduce the size of the feature map. For example, the transition layers 313, 315, and 317 may reduce the size of the feature map of features extracted by using the dense layer.

The classification layer 319 according to the embodiment may output pulmonary congestion state information based on the extracted features. The pulmonary congestion state information may include the binary classification value 320 including at least one of a probability value corresponding to an improved state of pulmonary congestion and a probability value corresponding to a state in which the user's pulmonary congestion has not improved.

FIG. 4 is a view for describing the vision transformer according to the embodiment outputting binary classification values.

Referring to FIG. 4, an apparatus for determining a state of pulmonary congestion (for example, pulmonary congestion state determination apparatus 100 of FIG. 1) may determine a pulmonary congestion state by using a vision transformer 430 as a pulmonary congestion state determination model. The vision transformer 430 according to the embodiment may include a projection unit 431 for converting the dimension of a feature vector of a partial spectrogram, a transformer encoder 432, and a classification layer 433. Each of the projection unit 431, the transformer encoder 432, and the classification layer 433 that are included in the vision transformer 430 may be one or more.

The data preprocessing unit according to the embodiment (for example, data preprocessing unit 120 of FIG. 1) may segment the mel-spectrogram 250 into a plurality of partial spectrograms 420 (or patches) by using cropping 410. The data preprocessing unit can input at least one of the segmented partial spectrograms 420 to a vision transformer 430.

According to the embodiment, the projection unit 431 may perform vector projection to transform the dimension of the feature vectors of the partial spectrograms 420. For example, the projection unit 431 may transform the dimension of the feature vectors of the partial spectrograms 420 into a dimension that may be processed by the transformer encoder 432.

The transformer encoder 432 according to the embodiment may calculate attention of vector-projected partial spectrograms. The transformer encoder 432 may calculate attention by performing normalization and multi-head attention on the feature vectors of the vector-projected partial spectrograms and passing the calculated attention through a fully connected layer.

The classification layer 433 according to the embodiment may output pulmonary congestion state information based on the calculated attention. The pulmonary congestion state information output by the classification layer 433 may include the binary classification value 440 including at least one of a probability value corresponding to an improved state of pulmonary congestion and a probability value corresponding to a state in which the user's pulmonary congestion has not improved.

In the embodiment, the pulmonary congestion detection model may be trained by using a machine learning process. The machine learning process enables the pulmonary congestion detection model to recognize input data patterns and make predictions on its own using given data. The machine learning process may include the following operations: (1) data preparation, (2) model initialization, (3) forward computation, (4) loss calculation, (5) backpropagation, and (6) parameter update. The data preparation process collects and preprocesses the training data from which the pulmonary congestion detection model will learn. The preprocessing process includes refining the training data and, when necessary, performing tasks such as standardization, normalization, and feature selection to transform the training data into a format suitable for the pulmonary congestion detection model. The model initialization process sets the initial parameters of the pulmonary congestion discrimination model. For example, when the pulmonary congestion discrimination model is a neural network, this may include initializing the weights and biases. The forward computation process inputs the prepared training data into the pulmonary congestion discrimination model to calculate the predicted value for estimating pulmonary congestion status. During this process, the pulmonary congestion discrimination model processes the training data using its current parameters and generates the predicted value as the output. The loss computation process calculates the difference between the predicted value of the pulmonary congestion discrimination model and the actual correct answer (label) using a loss function. A loss function is a function that allows the user to calculate a value that indicates how accurate (or inaccurate) the predictions of a pulmonary congestion detection model are. The backpropagation process is the process of adjusting the parameters of the pulmonary congestion detection model to reduce the loss derived from the loss function. The backpropagation algorithm determines the values of the loss function (gradient) to calculate how much each parameter of the pulmonary congestion detection model contributed to the loss, and based on this value, the parameters of the pulmonary congestion detection model may be updated. The parameter update process is the process of updating the parameters of the pulmonary congestion detection model by using the calculated gradient. In the parameter updating, gradient descent or a variant thereof may generally be used. Through this process, a pulmonary congestion classification model may be trained to make increasingly accurate predictions. The above processes (forward propagation, loss calculation, backpropagation, and parameter updating) may be repeatedly performed for a large amount of training data, and training may be conducted over multiple iterations until the pulmonary congestion classification model is sufficiently trained. Through such a training process, the pulmonary congestion classification model may learn patterns from given data and acquire an ability to make predictions on new data.

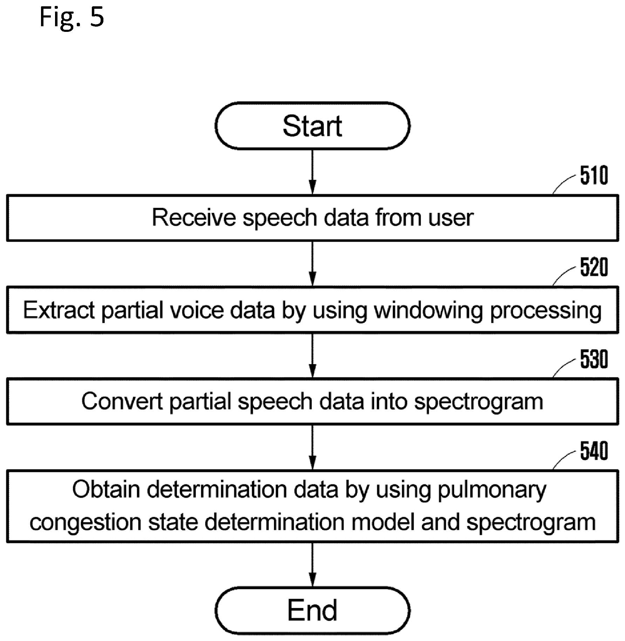

FIG. 5 is a flowchart for describing the operation of a method for determining a state of pulmonary congestion according to an embodiment. The operation of the method for determining a state of pulmonary congestion may be performed by the apparatus for determining a state of pulmonary congestion.

In operation 510, the apparatus for determining a state of pulmonary congestion (for example, pulmonary congestion state determination apparatus 100 of FIG. 1) may receive speech data from a user. The apparatus for determining a state of pulmonary congestion may receive the speech data from the user by using a data reception unit (for example, data reception unit 110 of FIG. 1) of FIG. 1. For example, the apparatus for determining a state of pulmonary congestion may receive speech data from a patient suffering from pulmonary congestion due to acute heart failure.

In operation 520, the apparatus for determining a state of pulmonary congestion may extract partial speech data by using windowing processing. For example, the apparatus for determining a state of pulmonary congestion may extract partial speech data corresponding to a portion of the speech data from the speech data by using windowing processing in units of 1 second.

In operation 530, the apparatus for determining a state of pulmonary congestion may convert partial speech data into a spectrogram. For example, the spectrogram may be a Mel-spectrogram, which is a graph in which the user's speech data is converted into frequency information corresponding to the speech data by Fourier transform and the scale of the frequency information is converted into a Mel-scale.

In operation 540, the apparatus for determining a state of pulmonary congestion may obtain determination data by using a pulmonary congestion state determination model and a spectrogram. The apparatus for determining a state of pulmonary congestion may obtain determination data including pulmonary congestion state information of a user using a neural network-based pulmonary congestion state determination model and a spectrogram. The spectrogram is input into the pulmonary congestion state determination model, and at least one of user information and blood test information of the user can be further input. The user information is personal information about the user by using the apparatus for determining a state of pulmonary congestion and may include, for example, age, gender, medical history, and lifestyle information. The blood test information is medical information about the user obtained by testing the user's blood and may include, for example, BNP, NT-proBNP, and ST2.

The pulmonary congestion state determination model may include, for example, a resnet (for example, resnet-50, resneXt), a dense network 310 (for example, densenet), a convnet (for example, ConvnetXt), an efficientnet (for example, efficientnet-b7), and/or a vision transformer (ViT).

According to the embodiment, a dense network (for example, dense network 310 of FIG. 3) may be a dense network including a convolution layer, a dense layer, a transition layer, and a classification layer. A pulmonary congestion detection device may extract hidden features through skip-connections of the dense network and determine pulmonary congestion state information by using the extracted hidden features. The pulmonary congestion state information output by the dense network according to the embodiment may include at least one of a probability value corresponding to an improved state of the user's pulmonary congestion and a probability value corresponding to a non-improved state of the user's pulmonary congestion. Through the pulmonary congestion state information, it may be possible to estimate whether and to what extent the user's pulmonary congestion has improved from the user's speech data.

The pulmonary congestion state determination model according to the embodiment may be a vision transformer (for example, vision transformer 430 in FIG. 5). The vision transformer may include a projection unit that transforms the dimension of the feature vector of the spectrogram, a transformer encoder, and a classification layer.

The apparatus for determining a state of pulmonary congestion may segment a spectrogram and input it to a vision transformer. For example, the apparatus for determining a state of pulmonary congestion may segment a spectrogram into multiple partial spectrograms (or patches) and input at least one of the segmented partial spectrograms to a vision transformer.

The apparatus for determining a state of pulmonary congestion according to an embodiment may perform vector projection on a segmented partial spectrogram through a vision transformer, calculate attention of the vector-projected partial spectrogram, and determine pulmonary congestion state information based on the calculated attention. The pulmonary congestion state information determined by the vision transformer may include at least one of a probability value corresponding to an improved state and a probability value corresponding to a state in which the user's pulmonary congestion is not improved. Through the pulmonary congestion state information, it may be possible to estimate whether the user's pulmonary congestion has improved and by how much it has improved from the user's speech data.

The method according to the embodiment may be implemented in the form of program commands that can be executed through various computer means and recorded on a computer-readable medium. The computer-readable medium may include program commands, data files, data structures, or the like, alone or in combination. The program commands recorded on the medium may be those specially designed and configured for the embodiment or may be those known and available to those skilled in the art of computer software. Examples of the computer-readable recording medium include magnetic media such as hard disks, floppy disks, and magnetic tapes, optical media such as compact disc read-only memories (CD-ROMs) and digital versatile discs (DVDs), magneto-optical media such as floptical disks, and hardware devices specially configured to store and execute program commands, such as ROMs, random access memories (RAMs), and flash memories. Examples of the program commands include not only machine language codes generated by a compiler, but also high-level language codes that can be executed by a computer using an interpreter, etc. The hardware devices described above may be configured to operate as one or more software modules to perform the operations of the embodiment, and vice versa.

Software may include a computer program, code, instructions, or a combination of one or more of these and may configure a processing device to operate as desired or may independently or collectively command the processing device. The software and/or data may be permanently or temporarily embodied in any type of machine, component, physical device, virtual equipment, computer storage medium, or device for interpretation by the processing device or for providing instructions or data to the processing device. The software may also be distributed over networked computer systems and stored or executed in a distributed manner. The software and data may be stored on one or more computer-readable recording media.

The term "module" as used in the present document may include a unit implemented in hardware, software, or firmware and may be used interchangeably with terms such as logic, logic block, component, or circuit. A module may be an integral component, or a minimum unit or part of such a component that performs one or more functions. For example, according to one embodiment, a module may be implemented in the form of an application-specific integrated circuit (ASIC).

The term "unit" as used in the present document refers to a software or hardware component, such as a field-programmable gate array (FPGA) or ASIC, that performs certain functions. However, "unit" is not limited to software or hardware. The "unit" may be configured to reside on an addressable storage medium and may be configured to execute one or more processors. For example, the "unit" may include components such as software components, object-oriented software components, class components, and task components, as well as processes, functions, attributes, procedures, subroutines, segments of program code, drivers, firmware, microcode, circuitry, data, databases, data structures, tables, arrays, and variables. The functionality provided in the components and "units" may be combined into a smaller number of components and "units" or further separated into additional components and "units." Furthermore, the components and "units" may be implemented to execute one or more CPUs within an apparatus or a secure multimedia card. Additionally, 'unit' may include one or more processors.

Although the embodiments described above have been described with limited drawings, those skilled in the art will appreciate that various technical modifications and variations may be applied based on the described embodiments. For example, appropriate results may still be achieved even when the described techniques are performed in a different order than described, and/or components of the described systems, structures, devices, circuits, and the like are combined or combined in a different manner than described or are replaced or substituted with other components or equivalents.

Therefore, other implementations, other embodiments, and equivalents to the claims also fall within the scope of the claims described below.

## Claims

1. An apparatus for determining a state of pulmonary congestion by using artificial intelligence analysis, the apparatus comprising:
a data reception unit configured to receive speech data from a user;
a data preprocessing unit configured to extract partial speech data corresponding to a portion of the received speech data by using windowing processing and to convert the extracted partial speech data into a spectrogram; and
a pulmonary congestion state determination unit configured to output determination data including pulmonary congestion state information of the user by using a neural network-based pulmonary congestion state determination model and the spectrogram.

2. The apparatus of claim 1, wherein the pulmonary congestion state determination model is a dense network including a convolution layer, a dense layer, a transition layer, and a classification layer.

3. The apparatus of claim 2, wherein the pulmonary congestion state determination model is a dense network and is configured to extract hidden features through skip-connections of the dense network and to determine the pulmonary congestion state information by using the extracted hidden features.

4. The apparatus of claim 1, wherein an output of the pulmonary congestion state determination model includes at least one of a probability value corresponding to a state in which pulmonary congestion of the user has improved and a probability value corresponding to a state in which pulmonary congestion of the user has not improved.

5. The apparatus of claim 1, wherein the pulmonary congestion state determination model is a vision transformer, and
the pulmonary congestion state determination model includes a projection unit configured to convert a dimension of a feature vector of the spectrogram, a transformer encoder, and a classification layer.

6. The apparatus of claim 5, wherein the data preprocessing unit is configured to divide the spectrogram into a plurality of partial spectrograms and to input at least one of the divided partial spectrograms into the vision transformer.

7. The apparatus of claim 5, wherein the vision transformer is configured to perform vector projection on the divided partial spectrogram, calculate attention of the vector-projected partial spectrogram, and determine pulmonary congestion state information based on the calculated attention.

8. The apparatus of claim 1, wherein the speech data from the user is speech data from a patient suffering from pulmonary congestion due to acute heart failure.

9. The apparatus of claim 1, wherein the spectrogram is a mel-spectrogram represented as a graph in which speech data of the user is transformed into frequency information corresponding to the speech data by a Fourier transform, and a scale of the frequency information is converted into a mel-scale.

10. The apparatus of claim 1, wherein the spectrogram is input to the pulmonary congestion state determination model, and at least one of user information of the user and blood test information of the user is further input to the pulmonary congestion state determination model.

11. A method for determining a state of pulmonary congestion performed by an apparatus for determining a state of pulmonary congestion, the method comprising:
receiving speech data from a user by using a data reception unit;
extracting partial speech data corresponding to a portion of the received speech data by using windowing processing and converting the extracted partial speech data into a spectrogram; and
obtaining determination data including pulmonary congestion state information of the user by using a neural network-based pulmonary congestion state determination model and the spectrogram.

12. The method of claim 11, wherein the pulmonary congestion state determination model includes a convolution layer, a dense layer, a transition layer, and a classification layer.

13. The method of claim 12, wherein the pulmonary congestion state determination model is a dense network, and hidden features are extracted through skip-connections of the dense network, and the pulmonary congestion state information is determined by using the extracted hidden features.

14. The method of claim 11, wherein an output of the pulmonary congestion state determination model includes at least one of a probability value corresponding to a state in which pulmonary congestion of the user has improved and a probability value corresponding to a state in which pulmonary congestion of the user has not improved.

15. The method of claim 11, wherein the pulmonary congestion state determination model is a vision transformer, and
the pulmonary congestion state determination model includes a projection unit configured to convert a dimension of a feature vector of the spectrogram, a transformer encoder, and a classification layer.

16. The method of claim 15, wherein the spectrogram is divided into a plurality of partial spectrograms, and at least one of the divided partial spectrograms is input to the vision transformer.

17. The method of claim 15, wherein the vision transformer performs vector projection on the divided partial spectrogram, calculates attention of the vector-projected partial spectrogram, and determines pulmonary congestion state information based on the calculated attention.

18. The method of claim 11, wherein the speech data from the user is speech data of a patient suffering from pulmonary congestion due to acute heart failure.

19. The method of claim 11, wherein the spectrogram is a mel-spectrogram represented as a graph in which speech data of the user is transformed into frequency information corresponding to the speech data by a Fourier transform, and a scale of the frequency information is converted into a mel-scale.

20. The method of claim 11, wherein the spectrogram is input to the pulmonary congestion state determination model, and at least one of user information of the user and blood test information of the user is further input to the pulmonary congestion state determination model.
